# EUROPÄISCHE PATENTSCHRIFT

(11) **EP 0 415 263 B1**
(45) Veröffentlichungstag und Bekanntmachung des Hinweises auf die Patenterteilung: **15.06.1994**
(21) Anmeldenummer: 90116139.8
(22) Anmeldetag: 23.08.1990
(51) Int. Cl.: C07D 265/30, A61K 9/107, A61K 47/22, C07D 211/38, C07D 207/10, C07D 401/06, C25B 3/08

(54) **Stabilisierung von Perfluorcarbonemulsionen und als emulsionsstabilisierende Zusätze verwendbare perfluorierte heterocyclische Verbindungen**
Stabilisation of perfluorocarbon emulsions and perfluorinated heterocyclic compounds usable as emulsion stabilising additives
Stabilisation d'émulsions perfluorocarbonées et composés perfluorés hétéricycliques utilisables comme additifs pour la stabilisation d'émulsions

(30) Priorität: 30.08.1989 DE 3928692; 15.12.1989 DE 3941514
(43) Veröffentlichungstag der Anmeldung: 06.03.1991
(73) Patentinhaber: Kali-Chemie Aktiengesellschaft, D-30173 Hannover (DE)
(72) Erfinder: Meinert, Hasso, D-7900 Ulm (DE); Mader, Jürgen, D-7900 Ulm (DE); Fackler, Rudolf, D-7913 Senden (DE); Reuter, Peter, D-7900 Ulm-Lehr (DE); Röhlke, Wolfgang, D-7900 Ulm-Mähringen (DE); Gennies, Monika, D-7900 Ulm (DE); Bär, Joachim, D-7900 Ulm (DE)
(74) Vertreter: Lauer, Dieter, Dr.

(56) Entgegenhaltungen:
- GB-A- 2 171 330
- US-A- 3 956 293
- CHEMICAL ABSTRACTS, Band 109, Nr. 22, 28. November 1988, Seite 655,Zusammenfassung Nr. 199847d, Columbus, Ohio, US; E. HAYASHI et al.:
- "Preparation of perfluorodiamines by electrochemical fluorination",& NAGOYA KOGYO GIJUTSU SHIKENSHO HOKOKU1988, 37(3), 54-61

## Beschreibung

Die vorliegende Erfindung betrifft die Verwendung einer Gruppe von perfluorierten heterocyclischen Verbindungen, welche zwei durch eine Perfluoroalkylenkette verbundene Heterocyclen enthalten, als emulsionsstabilisierende Zusätze in medizinisch verwendbaren wäßrigen Emulsionen von zur Verwendung in Blutersatzmitteln geeigneten gegebenenfalls Heteroatome enthaltenden Perfluorkohlenstoffen.

Ferner betrifft die vorliegende Erfindung zur Herstellung von medizinisch verwendbaren wäßrigen Emulsionen geeignete Gemische von physiologisch verträglichen gegebenenfalls Heteroatome enthaltenden Perfluorkohlenstoffen, welche zur Verwendung als Sauerstoffüberträger in Blutersatmitteln geeignete gegebenenfalls Heteroatome enthaltende Perfluorkohlenstoffe und emulsionsstabilisierende Zusätze der vorgenannten Gruppe von perfluorierten heterocyclischen Verbindungen enthalten, sowie medizinisch verwendbare wäßrige Emulsionen dieser Gemische, sowie neue als emulsionsstabilisierende Bestandteile von Perfluorcarbonemulsionen geeignete perfluorierte heterocyclische Verbindungen.

Als Perfluorkohlenstoffe oder Perfluorcarbone werden gesättigte perfluorierte organische Verbindungen bezeichnet, welche aus Kohlenstoff und Fluor bestehen und gegebenenfalls noch Heteroatome, beispielsweise Stickstoff oder Sauerstoff, in der Kohlenstoffkette enthalten können. Im folgenden werden mit dem Begriff "Perfluorcarbone" gegebenenfalls Heteroatome enthaltende Perfluorokohlenstoffverbindungen bezeichnet. Derartige Verbindungen sind bekannt, z.B. aus den europäischen Patentanmeldungen, Veröffentlichungsnummer 77 114, 99 652 und 151 697. Die Perfluorcarbonmoleküle sind durch eine einheitliche Schale von Fluoratomen hervorragend abgeschirmt. Daher sind Perfluorcarbone chemisch und physiologisch außerordentlich inert, also ungiftig. Wegen ihrer extrem geringen intermolekularen Kräfte haben Perfluorcarbone einem im Verhältnis zu ihren Molekülmassen tiefen Siedepunkt und eine außerordentlich geringe Oberflächenspannung. Aus den sehr schwachen intermolekularen Kräften resultiert auch die Fähigkeit der Perfluorcarbone große Mengen von Gasen, beispielsweise Sauerstoff und Kohlendioxid zu lösen. Aufgrund dieser Eigenschaften, insbesondere der Fähigkeit, Sauerstoff physikalisch zu lösen und zu transportieren, haben Perfluorcarbone in der Medizin Anwendung gefunden zur Herstellung von wäßrigen Sauerstoff transportierenden Perfluorcarbonemulsionen, welche zum Beispiel als Blutersatzmittel oder als Medien für Organperfusion und -lagerung in der Transplantationschirurgie eingesetzt werden können.

Perfluorcarbone sind hydrophobe mit Wasser nicht mischbare Substanzen. Sie können daher nicht als solche, sondern nur in Form von physiologisch verträglichen wäßrigen Emulsionen in den Blutkreislauf eingeführt werden. Derartige Emulsionen werden bekannterweise mit Hilfe eines physiologisch verträglichen Emulgators hergestellt und stellen Emulsionen vom Öl-in-Wasser-Typ dar.

Neben einer guten physiologischen Verträglichkeit, z.B. dem normalen Blut ähnlichen osmotischen und onkotischen (kolloidosmotischen) Drucken, Fließeigenschaften und pH-Konstanz, und einer guten Sauerstofflösekapazität ist es für als Blutersatzmittel verwendbare Emulsionen wichtig, daß sie eine geeignete Verweildauer im Blutkreislauf aufweisen und anschließend möglichst vollständig und ohne übermäßige Retention in Organen wieder ausgeschieden werden. Als günstig werden Verweilhalbwertzeiten im Körper von ca. einer Woche bis zu einem Monat, insbesondere von ca. 2 - 4 Wochen, angesehen. Ferner ist es wichtig, daß die Emulsionen eine ausreichende Stabilität gegen ein Wachstum der Ölphasenpartikel aufweisen.

Perfluorcarbone werden dank ihrer chemischen Inertheit im Körper praktisch nicht metabolisiert und werden in unveränderter Form mit der Atemluft oder durch die Haut ausgeschieden. Die Ausscheidungsrate einzelner Perfluorcarbone ist sehr unterschiedlich und kann von wenigen Tagen bis zu einigen Jahren variieren. Die Ausscheidungsrate der einzelnen Substanzen hängt dabei stark von deren Dampfdruck sowie auch von ihrer Löslichkeit in niedrigpolaren Medien ab, da die Durchtrittsgeschwindigkeit der Perfluorcarbone durch die alveolaren Membranen von diesen Parametern abhängig ist.

Als Maß für die Lipophilie der Perfluorcarbone kann die kritische Lösungstemperatur in n-Hexan (=CTSH), das ist die Temperatur, bei welcher sich das betreffende Perfluorcarbon in einer gleichen Menge n-Hexan löst, dienen. Die Bestimmung der CTSH eines Perfluorcarbons ist daher eine in-vitro-Testmethode, welche ein gutes Indiz für die Verweildauer des Perfluorcarbons im Körper darstellt.

Für eine befriedigende Exhalationsrate sind eine ausreichende Lipophylie und ein ausreichend hoher Dampfdruck vorteilhaft.

Der Hauptgrund des Tröpfchenwachstums in Emulsionen liegt in dem Zusammenfließen (Coalescenz) mehrerer Tröpfchen. Dieses kann im allgemeinen durch die Verwendung von Emulgatoren, welche eine Barriere an der Öl-Wasser-Grenzfläche bilden und die Grenzflächenspannung heruntersetzen, zurückgedrängt werden. Eine weitere weniger auffällige Ursache der Alterung von Emulsionen unter Partikelwachstum kann in einer molekularen Diffusion liegen, welche als Ostwald Ripening bekannt ist. Hierbei diffundieren Moleküle aus kleinen Tröpfchen der Ölphase durch die wäßrige Phase in größere Tröpfchen der Ölphase. Diese molekulare Diffusion kann auch in Anwesenheit von gegen die Koaleszenz wirksamen Emulgatoren stattfinden. Das Phänomen der molekularen Diffusion tritt umso stärker auf, je höher der Dampfdruck der betreffenden Perfluorcarbone ist.

Als sauerstoffübertragende Komponenten von als Blutersatzmitteln verwendbaren Emulsionen werden bevorzugt Perfluorcarbone eingesetzt, welche eine Verweilhalbwertzeit im Körper von einer Woche bis einem Monat besitzen. Eine Zufuhr größerer Mengen von Substanzen mit höheren Verweilhalbwertzeiten ist nicht zweckmäßig, da sie zu unerwünschter langandauernder Retention der Substanzen im Körper führen kann. Perfluorcarbone mit Verweilhalbwertzeiten von unter einer Woche sind ebenfalls nicht zweckmäßig. Perfluorcarbone mit geeigneten Verweilhalbwertzeiten im Körper sind im allgemeinen bei Körpertemperatur flüssige Perfluorcarbone, z.B. Substanzen mit einem Siedepunktbereich von 130 - 200 °C und mit einem entsprechend hohen Dampfdruckbereich und Molekulargewichten im Bereich von 450 - 600, insbesondere 460 - 550. Diese Substanzen haben jedoch den Nachteil, daß ihre Emulsionen eine starke Tendenz zu Partikelwachstum durch molekulare Diffusion zeigen.

Perfluorodecalin ist ein derartiges Perfluorcarbon, welches auf Grund seiner guten Sauerstofftransportfähigkeit, seiner Ungiftigkeit und seiner befriedigenden Ausscheidungsrate bereits in als Blutersatzmittel dienenden Emulsionen klinisch eingesetzt wird. Leider liefert diese Substanz keine bei Raumtemperatur über längere Zeit stabilen Emulsionen. Es wurde bereits versucht, stabilere Emulsionen zu erzielen, in denen anstelle von Perfluorodecalin, welches nur aus Kohlenstoff und Fluor aufgebaut ist, ein Perfluorcarbongemisch aus Perfluorodecalin und eine ein Stickstoffatom enthaltenden nicht cyclischen Perfluorcarbon, welches auf Grund des in dem Molekül enthaltenen N-Atom eine gewisse Polarität aufweist, einzusetzen, beispielsweise Perfluorotripropylamin oder Perfluorotributylamin. Zur Erzielung einer nutzbringenden Verbesserung der Emulsionsstabilität war es notwendig, ca. ein Drittel des Perfluorodecalins durch Perfluorotripropylamin zu ersetzen. So stellt das als Blutersatzmittel im Handel befindliche Fluosol-DA^{R} 20 % (Hersteller Green Cross Corporation) eine wäßrige Emulsion dar, welche 14 % (G/V) Perfluorodecalin und 6 % (G/V) Perfluortripropylamin sowie physiologisch verträgliche Emulgatoren und weitere Hilfsstoffe enthält. Auch dieses Produkt weist noch keine zufriedenstellende Langzeitstabilität auf. Ferner ist der hohe Gehalt an polarerem, höhersiedendem stickstoffhaltigem Perfluorocarbon mit einer ungünstig hohen Verweilhalbwertzeit im Körper von ca. 65 Tagen nachteilig.

Der vorliegenden Erfindung liegt die Aufgabe zugrunde, zur Herstellung von für medizinische Zwecke, insbesondere als Blutersatzmittel, geeigneten wäßrigen Perfluorcarbonemulsionen mit verbesserter Stabilität verwendbare Perfluorcarbongemische zu entwickeln.

Es wurde nun gefunden, daß das auf molekularer Diffusion beruhende unerwünschte Wachstum der Perfluorcarbonphasenpartikel in wäßrigen Emulsionen von als Sauerstoffüberträger in Blutersatzmitteln verwendbaren Perfluorcarbonen sich hemmen läßt durch Zusatz von Verbindungen einer Gruppe von Perfluorcarbonen, welche sich in ihrer chemischen Struktur dadurch auszeichnen, daß sie zwei (je ein tertiär an 3 Kohlenstoffatome gebundenes Stickstoffatom enthaltende) perfluorierte Ringe enthalten,
Welche durch eine Perfluoralkylenkette verbunden sind und daß der Zusatz dieser Verbindungen zu Perfluorcarbonen zu einer guten Emulgierbarkeit der so erhaltenen Perfluorcarbongemische und einer hohen Stabilität von wäßrigen Emulsionen dieser Gemische führt.

Aus einer Arbeit von Hayashi et al. (Chem. Express 1988, 3, 191-194, siehe chem. Abstr. Vol 110, 75255k (1989)) sind perfluorierte heterocyclische Äthylen- und Propylendiamine bekannt, welche als inerte Flüssigkeiten zur Anwendung in der Elektronik-Industrie vorgeschlagen werden. Aus US 3 956 293 ist das Perfluoro-N,N'-hexamethylen-bis-morpholin als Ausgangsprodukt zur Herstellung einer entsprechenden sulfofluorierten als chemisches Zwischenprodukt einsetzbaren Substanz bekannt. Für diese Verbindungen ist jedoch keinerlei Anwendung in medizinisch verwendbaren wäßrigen Emulsionen bekannt.

Gegenstand der vorliegenden Erfindung ist die Verwendung von perfluorierten heterocyclischen Verbindungen der allgemeinen Formel I,
(siehe Formel I)
worin
- n: für 2-8 steht,
- X: eine -CF₂-O-CF₂-Gruppe, eine -CF₂-CF₂-CF₂-Gruppe oder eine -CF(CF₃)-CF₂-Gruppe darstellt, und
- Y: eine -CF₂-O-CF₂-Gruppe, eine -CF(CF₃)-CF₂-Gruppe oder eine -CF₂-CF₂-CF₂-Gruppe darstellt,
oder deren Gemischen als emulsionsstabilisierende Zusätze in medizinisch verwendbaren wäßrigen Emulsionen von zur Verwendung in Blutersatzmitteln geeigneten gegebenenfalls Heteroatome enthaltenden Perfluorkohlenstoffen.

Hierzu eignen sich vorzugsweise solche Verbindungen der Formel I, worin n 3-6, insbesondere 3 oder 4 bedeutet.

Gegenstand der Erfindung sind ferner zur Herstellung von medizinisch verwendbaren wäßrigen Perfluorcarbonemulsionen mit verbesserter Stabilität geeignete Perfluorcarbongemische, welche zur Verwendung als Sauerstoffüberträger in Blutersatzmitteln geeignete gegebenenfalls Heteroatome enthaltende Perfluorcarbone mit einem Zusatz an Verbindungen der Formel I enthalten, und diese Perfluorcarbongemische enthaltende wäßrige Emulsionen.

Um eine wirksame Stabilitätsverbesserung der Emulsionen zu erzielen, können Mengen von 1 - 20, zweckmäßigerweise 2,5 bis 10 Gew.-% bezogen auf den Gesamtperfluorocarbongehalt der Emulsionen eingesetzt werden. Es sind also Mengen von 0,5 - 2 g der Verbindungen der Formel I in 100 ml Emulsion ausreichend, um eine wirksame Stabilitätsverbesserung der Emulsionen durch Hemmung des auf molekularer Diffusion beruhenden Partikelwachstums zu erzielen.

Es wurde ferner gefunden, daß durch den erfindungsgemäßen Zusatz von Verbindungen der Formel I zu wäßrigen Perfluorcarbonemulsionen die Grenzflächenspannung zwischen Perfluorcarbonphase und Wasser herabgesetzt wird.

Auf Grund dieses Phänomens wird angenommen, daß die Verbindungen der Formel I sich an der Oberfläche der Perfluorcarbonpartikel ansammeln und dort ihre diffusionshemmende Wirkung entfalten.

Die Verbindungen der Formel I eignen sich zur Stabilisierung von wäßrigen Emulsionen von solchen Perfluorcarbonen, welche auf Grund ihrer physikalischen Eigenschaften in wäßrigen Emulsionen zu Partikelwachstum durch molekulare Diffusion neigen. Hierzu gehören die als Sauerstoffüberträger in Blutersatzmitteln geeigneten Perfluorcarbone, welche sich durch Verweilhalbwertzeiten im Körper von zwischen einer Woche und einem Monat, vorzugsweise 2 bis 3 Wochen, auszeichnen. Hierzu gehören Perfluorcarbone, welche bei Normaltemperatur flüssig bis wachsartig sind und deren Siedepunkte im Bereich von 130 bis 200, insbesondere von 130 bis 180 °C, liegen. Derartige Perfluorcarbone können Molekulargewichte im Bereich von 450 - 600, vorzugsweise 460 - 550, besitzen. Zu diesen Perfluorcarbonen gehören nur aus Fluor und Kohlenstoff bestehende cyclische und offenkettige oder durch Perfluoralkylreste substituierte cyclische Verbindungen sowie auch Heteroatome, beispielsweise 1 - 4 Heteroatome, insbesondere Stickstoff und/oder Sauerstoff, enthaltende cyclische gegebenenfalls durch Perfluoralkylreste substituierte Verbindungen. Cyclische Verbindungen können mono- oder bicyclische Struktur bezitzen. Als bevorzugtes Beispiel einer heteroatomfreien Substanz sei das Perfluorodecalin (= Perfluorodecahydronaphthalin) genannt. Als Beispiele von heteroatomhaltigen Substanzen seien das Perfluorcyclohexylmorpholin und dessen Gemische mit Perfluoro-n-hexylmorpholin genannt, welche in einer Parallelpatentanmeldung beschrieben werden und durch elektrochemische Fluorierung von durch Umsetzung von Morpholin mit Cyclohexanon erhältlichem Morpholinocyclohexen-(1) unter den nachstehend zur Herstellung der Verbindungen der Formel I angegebenen Bedingungen erhalten werden können.

Die Erfindung betrifft ferner neue perfluoriete heterocyclische Verbindungen der allgemeinen Formel I′,
(siehe Formel I′)
worin
- n': für 4 steht,
- X: eine -CF₂-O-CF₂-Gruppe, eine -CF₂-CF₂-CF₂-Gruppe oder eine -CF(CF₃)-CF₂-Gruppe darstellt, und
- Y: eine -CF₂-O-CF₂-Gruppe, eine -CF(CF₃)-CF₂-Gruppe oder eine -CF₂-CF₂-CF₂-Gruppe darstellt,
sowie deren Herstellung.

Die neuen Verbindungen der Formel I' besitzen ein gutes Lösungsvermögen für Gase wie beispielsweise Sauerstoff und Kohlendioxid, sind physiologisch inert und zeichnen sich durch eine besonders gute Emulgierbarkeit und eine hohe Stabilität der sie enthaltenden wäßrigen Emulsionen aus. Aufgrund ihrer Eigenschaften sind die erfindungsgemäßen Verbindungen beispielsweise geeignet als Zusätze zur Herstellung von medizinisch verwendbaren wäßrigen Emulsionen, welche beispielsweise als Blutersatzmittel oder Perfusionsmittel Verwendung finden.

Ferner sind die Verbindungen auch zur Anwendung in anderen technischen Gebieten, in welchen ungiftige und chemisch inerte flüssige oder wachsartige Substanzen benötigt werden, bzw. inerte Substanzen mit einem Lösevermögen für Gase benötigt werden, geeignet. So eignen sich die Verbindungen der Formel I' und ihre Gemische beispielsweise als inerte Kühlmittel, Schmiermittel, Sperr-und Hydraulikflüssigkeiten, Isoliermedien in der Elektrotechnik, sowie Mittel zum Dampfphasenlöten bzw. als Zusätze zu Mitteln für die vorgenannten Zwecke. Verbindungen der Formel I' mit bei Raumtemperatur wachsartiger bis fester Konsistenz eignen sich als inerte temperaturbeständige Schmier-, Gleit- und Dichtungsmittel, welche über einen weiten Temperaturbereich einsetzbar sind. Aufgrund ihres Lösevermögens für Gase eignen sich die Verbindungen der Formel I und ihre Gemische als inertes Medium für die Diffusion von Gasen zwischen verschiedenen Phasen. Damit können die Verbindungen auch Verwendung finden bei Verfahren zur technischen Trennung von Gasen, beispielsweise der Dialysetrennung von Gasen, wobei die Verbindungen als inerte Austauschphase dienen können.

Verbindungen der Formel I können erhalten werden, indem man Verbindungen der allgemeinen Formel II
(s. Formel II)
worin
- n: obige Bedeutung besitzt,
- A: Sauerstoff oder eine -CH₂-Gruppe bedeutet, und
- B: Sauerstoff oder eine -CH₂-Gruppe bedeutet,
nach an sich bekannten Methoden fluoriert. So können die Verbindungen durch elektrochemische Fluorierung dargestellt werden, indem man Lösungen der Verbindungen der Formel II in flüssigem Fluorwasserstoff elektrolysiert. Zweckmäßig werden für das Verfahren Lösungen von 4 - 30, vorzugsweise 5 - 10 Gew.-% einer Verbindung der Formel II in flüssigem Fluorwasserstoff eingesetzt. Die Elektrolyse findet vorteilhaft bei Temperaturen zwischen -25 und +10, vorzugsweise -5 und +5 °C, einer Anodenstromdichte von 2 -30 mA/cm² und einer Zellspannung von 3 - 10, insbesondere 4 - 8 V, statt.

Zur weiteren Aufarbeitung wird das sich als schwere Phase am Boden der verwendeten Elektrolysezelle absetzende rohe Reaktionsprodukt abgetrennt und zur Zersetzung von allfälligen nur teilfluorierten Nebenprodukten einer Behandlung mit einer Alkali- oder Erdalkalimetallbase, insbesondere einem Alkali- oder Erdkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur unterworfen. Diese Verfahrensstufe kann unter an sich bekannten Reaktionsbedingungen, beispielsweise analog zu den in den europäischen Patentanmeldungen Veröffentlichungs-Nr. 99 652 und 151 697 beschriebenen Methoden, erfolgen. Als zweckmäßig erweist sich das Behandeln des rohen Reaktionsproduktes mit einer 6- bis 10-normalen wäßrigen Alkalimetallhydroxidlösung, insbesondere Kaliumhydroxidlösung, und einem niederen aliphatischen Amin bei erhöhter Temperatur, vorzugsweise Siedetemperatur des Reaktionsgemisches. So kann das Reaktionsgemisch beispielsweise für eine Dauer von mehreren Stunden bis zu 8 Tagen unter Rückfluß zum Sieden erhitzt werden. Als niedere aliphatiche Amine eignen sich niedere bei Raumtemperatur flüssige primäre oder sekundäre Amine oder Diamine, vorzugsweise sekundäre Amine wie beispielsweise Dialkylamine mit bis zu 5 Kohlenstoffatomen in ihren Alkylresten, Hexymethylendiamin oder auch cyclische Amine wie Piperidin. Vorzugsweise wird ein Dibutylamin wie beispielsweise Diisobutylamin eingesetzt.

Aus dem entstandenen Reaktionsgemisch können die Verbindungen der Formel I oder ihre Gemische auf an sich bekannte Weise beispielsweise durch fraktionierte Destillation oder durch präparative Gaschromatographie isoliert werden.

Die durch fraktionierte Destillation aus dem Reaktionsgemisch abgetrennten Verbindungen sind frei von nicht perfluorierten Produkten. Sie können jedoch noch ebenfalls perfluorierte und somit chemisch und physiologisch inerte Nebenprodukte enthalten. Insbesondere können sie solche perfluorierten Nebenprodukte enthalten, welche ein ähnliches Molekulargewicht wie das Hauptprodukt besitzen und damit im gleichen Temperaturbereich wie das Hauptprodukt sieden. Die Anwesenheit derartiger perfluorierter Nebenprodukte beeinträchtigt die erfindungsgemäße Verwendung der Verbindungen jedoch nicht, so daß die destillativ gereinigten Produkte im allgemeinen ohne weitere Reinigung verwendet werden können.

Unter den Bedingungen der elektrochemischen Perfluorierung von Verbindungen der Formel II findet in piperidinringhaltigen Verbindungen teilweise in den Piperidinringen eine Ringverengung statt, so daß neben Verbindungen der allgemeinen Formel I, worin X eine -CF₂-CF₂-CF₂-Gruppe bedeutet, in geringerem Ausmaß auch die dazu isomeren Verbindungen, worin X eine -CF(CF₃)-CF₂-Gruppe bedeutet, erhalten werden.

Diese Isomerengemische von Verbindungen der Formel I können in gleicher Weise wie reine Verbindungen der Formel I verwendet werden. Gewünschtenfalls können die Isomerengemische in ihre einzelnen Isomeren aufgetrennt werden. Zweckmäßigerweise wird die Isomerentrennung mittels Adsorption/Desorption an Molekularsieben, vorzugsweise Molekularsieben mit einer Porengröße von 5-6 Å, durchgeführt. Als Molekularsiebe eignen sich z.B. anorganische Alumosilikate, Zeolithe und Silicalite (= Siliciumdioxide mit geeigneter Porengröße). Bevorzugt werden Zeolithe eingesetzt. Anorganische Molekularsiebe eignen sich generell zur Trennung von erfindungsgemäßen nur unsubstituierte perfluorierte Ringe enthaltenden Verbindungen von Perfluoroalkylsubstituenten enthaltenden Nebenprodukten. Je nach Größe der Perfluoroalkylsubstituenten werden hierfür Molekularsiebe mit Porengrößen zwischen 5 und 6,5 Å gewählt. Eine Auftrennung von Isomerengemischen kann auch durch präparative Gaschromatographie in an sich bekannter Weise erfolgen.

Die Ausgangsverbindungen der Formel II sind bekannt oder können nach an sich bekannten oder analog zu an sich bekannten Methoden hergestellt werden.

Beispielsweise können die Ausgangsverbindungen der Formel II, erhalten werden, indem sekundäre Amine der allgemeinen Formel III
(s. Formel III)
worin A obige Bedeutung besitzt, mit Verbindungen der allgemeinen Formel IV
(s. Formel IV)
worin E Halogen, insbesondere Chlor oder Brom, oder die Hydroxylgruppe bedeutet und n obige Bedeutung besitzt, in an sich bekannter Weise umsetzt.

Verbindungen der Formel II können auch erhalten werden, indem man Dihalogenide der allgemeinen Formel V
(s. Formel V)
worin A und E obige Bedeutung besitzen, mit Diaminen der allgemeinen Formel VI
(s. Formel VI)
worin n obige Bedeutung besitzt, in an sich bekannter Weise umsetzt.

Die vorliegende Erfindung betrifft auch medizinisch verwendbare wäßrige Emulsionen. Die erfindungsgemäßen Emulsionen stellen Emulsionen vom Öl-in-Wasser-Typ dar, welche pro 100 ml Emuision 5 - 50, insbesondere 15 - 25, vorzugsweise ca. 20 g, an Perfluorcarbongemischen, worin 1 - 20, vorzugsweise 2,5 -10 Gew.-%, an Verbindungen der Formel I enthalten sind, und einen physiologisch verträglichen Emulgator enthalten. Es kann sich insbesondere als zweckmäßig erweisen, den Gemischen Verbindungen der nachfolgenden Beispiele 2b, 2c, 2i oder 2j zuzusetzen.

Als Emulgatoren eignen sich physiologisch verträgliche Emulgatoren, welche selbst untoxisch sind, keine Hämolyse verursachenden Eigenschaften haben und auchsonst keine Wechselwirkungen mit Komponenten des natürlichen Blutes eingehen und vollkommen aus dem Körper ausgeschieden oder unter Bildung untoxischer Metaboliten metabolisiert werden. Geeignete Emulgatoren sind zum Beispiel natürliche Phospholipide wie Eilecithine oder Sojalecithine, und Albumine. Ebenfalls geeignet sind physiologisch verträgliche nichtionische Emulgatoren vom Typ Äthylenoxid-Propylenoxid-Mischpolymere, beispielsweise Mischpolymere mit einem Molekulargewicht im Bereich von 8000 -8500. Derartige Emulgatoren sind als Handelsprodukte erhältlich, beispielsweise unter dem Warenzeichen Pluronic^{R}, Hersteller Wyandotte Chemicals Corp. Die Emulgatoren können in den erfindungsgemäßen Emulsionen in einer Konzentration von beispielsweise 2 - 7 g pro 100 ml Emulsion enthalten sein. Außerdem können die Emulsionen noch weitere in Blutersatzmitteln übliche Hilfs- und Zusatzstoffe enthalten, beispielsweise Salze und Substanzen, welche zur Einstellung eines physiologisch verträglichen pH-Wertes, und/oder osmotischen und onkotischen Druckes dienen.

Die erfindungsgemäßen Emulsionen können in an sich bekannter Weise mit üblichen Emulsionstechniken hergestellt werden. So kann die Emulgierung durch Ultraschall- und/oder Hochdruckhomogenisation erfolgen. Der Zusatz der heteroatomhaltigen Verbindungen der Formel I zu den Emulsionsansätzen verbessert nicht nur die Emulsionsstabilität, sondern erleichtert auch die Emulgierbarkeit des Gemisches. So kann eine Emulgierung zu stabilen Emulsionen in kurzer Zeit mit einfachen Geräten erfolgen.

Die erfindungsgemäßen Emulsionen besitzen vorzugsweise Kolloidteilchengrößen im Bereich von 50 - 300 nm und zeichnen sich durch eine hohe Langzeitstabilität aus.

Die erfindungsgemäßen Emulsionen können in der Medizin Anwendung finden als Sauerstoff transportierende Blutersatzmittel. Außerdem sind sie als Sauerstoff transportierende Perfusionslösungen, beispielsweise zum Schutze von freigelegten Organen in der Chirurgie, beispielsweise zum Schutze des Myokardium gegen Hypoxie in der Herzchirurgie, oder als Perfusions- und Transportflüssigkeiten in der Transplantationschirurgie einsetzbar. Ferner können die Emulsionen auch als Hilfsmittel in der Diagnostik, beispielsweise für die Ultrasonographie und ¹⁹F-NMR-Tomographie Einsatz finden. Auch in der Biotechnologie können erfindungsgemäße Emulsionen in Sauerstoff übertragenden Nährmedien, beispielsweise zur Züchtung von tierischen und pflanzlichen Zellen oder bei der Interferonsynthese Anwendung finden.

Die nachstehenden Beispiele sollen die Erfindung näher erläutern ohne jedoch ihren Umfang zu beschränken.

Als Elektrolysezellen für die elektrochemische Fluorierung werden Zellen mit Elektroden aus Nickel verwendet, welche entweder ein Fassungsvolumen von 300 ml und eine Anodenfläche von 475 cm² oder ein Fassungsvolumen von 960 ml und eine Anodenfläche von 1530 cm² besitzen. Die Zellen sind mit einem Rückflußkühler versehen, welcher auf einer Temperatur zwischen -15 und -20 °C gehalten wird.

### Beispiel 1:

Herstellung von einem Gemisch aus perfluoriertem 1,3-Dipiperidinopropan und den dazu isomeren perfluorierten 1-(3-Methylpyrrolidino)-3-piperidinopropan und 1,3-Di-(3-Methylpyrrolidino)-propan.

Eine 10%ige Lösung von 1,3-Dipiperidinopropan in vorgetrocknetem gekühlten flüssigen Fluorwasserstoff wurde in einer Elektrolysezelle bei einer Temperatur von +2 °C, einer Anodenstromdichte von 2,5 bis 25 mA/cm² und einer Zellspannung von 4,5 bis 7,0 V perfluoriert. Von Zeit zu Zeit wurden in flüssigem Fluorwasserstoff gelöstes Dipiperidinopropan und verbrauchter Fluorwasserstoff nachgefüllt, um ein kontinuierliches Arbeiten der Zelle zu ermöglichen. Das sich am Zellboden sammelnde rohe Reaktionsprodukt wurde von Zeit zu Zeit abgelassen. Zur Aufarbeitung wurde das Rohprodukt mit jeweils den gleichen Volumina einer wäßrigen 8-N Kaliumhydroxidlösung und Dibutylamin versetzt. Das Gemisch wurde 8 Tage am Rückfluß erhitzt. Anschließend wurde das Gemisch fraktionierend destilliert. Bei der Destillation wurde eine Hauptfraktion, welche bei 195 bis 203 °C siedete, erhalten. Diese Fraktion bestand aus dem im Titel angegebenen Isomerengemisch und konnte ohne weitere Auftrennung für die in der vorgehenden Beschreibung angegebenen Verwendungszwecke eingesetzt werden.

Eine gaschromatische Auftrennung des Gemisches ergab, daß dieses 58 % perfluoriertes 1,3-Dipiperidinopropan mit einem Siedepunkt 191 °C, 36 % perfluoriertes 1-(3-Methylpyrrolidino-3-piperidinopropan mit einem Siedepunkt von 189 °C und 6 % perfluoriertes 1,3-Di(3-methylpiperidino)propan mit einem Siedepunkt von 186 °C enthielt.

### Auftrennung des isomeren Gemisches:

Da das als Hauptkomponente in dem Gemisch enthaltene perfluorierte 1,3-Dipiperidinopropan und seine Isomeren wegen ihrer gleichen Molekulargewichte und damit ungefähr gleichen Siedepunkte destillativ schlecht voneinander zu trennen sind, wurde die Trennung durch fraktionierte Absorption an einer mit einem einem Zeolith-Molekularsieb mit einer Porengröße von 5 bis 6 Å gefüllten gaschromatographischen Trennsäule durchgeführt.

### Beispiel 2:

Analog zu den in dem vorstehenden Beispiel angegebenen Methoden wurden auch die in der folgenden Tabelle 1 angegebenen Verbindungen der Formel I durch elektrochemische Perfluorierung entsprechender Ausgangsverbindungen der Formel II erhalten.

Die Struktur der Verbindungen wurde durch Elementaranalyse, NMR-Spektroskopie, IR-Spektroskopie, Massenspektroskopie und Gaschromatographie verifiziert.

Das Sauerstofflösevermögen der in Tabelle 1 angegebenen Verbindungen der Formel I (=O₂-lös.) wurde wie folgt bestimmt:
Die Bestimmung wurde in einem mit einem Ventil für Sauerstoffzufuhr, einem Ventil zur Evakuierung und einem Druckmesser versehenen 2 l-Rundkolben mit einem zur Aufnahme des zu untersuchenden Perfluorcarbon geeigneten unteren Fortsatz, in welchen das Perfluocarbon durch ein Septum eingeführt werden kann, durchgeführt.

Die Apparatur wurde zunächst mit einer Wasserstrahlpumpe evakuiert, mit Sauerstoff gefüllt, nochmals evakuiert und wieder mit Sauerstoff bis zum Erreichen eines Innendruckes von 1013 mbar gefüllt. Sodann wurde das Perfluorcarbon durch ein Septum in den unteren Fortsatz eingeführt und dort 2 Stunden unter Sauerstoffatmosphäre gerührt. Die Temperatur wurde mit Hilfe eines Kryostaten auf 37 °C gehalten. Nach 2 Stunden wurde ein Teil des Perfluorcarbon entnommen und gaschromatographisch auf seinen Sauerstoffgehalt untersucht.

Die gaschromatische Untersuchung wurde mit einem Gaschromatographen CAP 12 der Fa. Gira, Frankreich, vorgenommen. Als Trägergas wurde Helium mit einem Fluß von 20 ml/min. eingesetzt. Als Trennsäule wurde eine Glassäule 2 m x 4 mm ⌀ gefüllt mit Molekularsieb 5 Å, 40-60 mesh, verwendet. Die Ofentemperatur betrug 100 °C, die Injektortemperatur 250 °C und die Detektortemperatur 200 °C. Das Probenvolumen betrug 30 - 50 »l.

**Tabelle 1**

| Bsp. Nr. | Substanz* Perfluor- | Mol-Gew. | Siedepunkt in °C | Ausbeute | O₂-lösl.** Vol % 37 °C |
|---|---|---|---|---|---|
| 2 a | Dimorpholinoethan | 560 | 162-166 (Fp.76°C) | 28 % | 44 |
| 2 b | Dimorpholinopropan | 610 | 181-183 (Fp.56°C) | 25 % | 43 |
| 2 c | Dimorpholinobutan | 660 | 196-200 (Fp.65°C) | 30 % | 41 |
| 2 d | Dimorpholinopentan | 710 | 214-216 (Fp.60°C) | 26 % | 41 |
| 2 e | Dimorpholinohexan | 760 | 223-226 (Fp.80°C) | 20 % | 40 |
| 2 f | Dimorpholinoheptan | 810 | 238-243 | 21 % | |
| 2 g | Dimorpholinooctan | 860 | 250-253 | 14 % | |
| 2 h | Dipiperidinoethan | 628 | 181-189 | 26 % | 43 |
| 2 i | Dipiperidinopropan | 678 | 196-205 | 41 % | 42 |
| 2 j | Dipiperidinobutan | 728 | 210-217 | 22 % | 42 |
| 2 k | Dipiperidinopentan | 778 | 220-229 | 42 % | 39 |
| 2 l | Dipiperidinohexan | 828 | 235-239 | 15 % | 38 |
| 2 m | Dipiperidinoheptan | 878 | 260-273 | 12 % | |
| 2 n | Dipiperidinooctan | 928 | 278-290 | 9 % | |

| | | | | | |
|---|---|---|---|---|---|
| *destillativ gewonnenes Produkt, enthält keine nicht perfluorierten Bestandteile, kann jedoch noch geringe Mengen perfluorierter Nebenprodukte enthalten | | | | | |
| ** bestimmt jeweils für das reine Hauptprodukt | | | | | |

Die verwendeten Ausgangsstoffe der Formel II wurden nach den folgenden allgemeinen Arbeitsvorschriften hergestellt:
A) Ausgehend von einem Dihalogenid der Formel V und einem Diamin der Formel VI.
   0,4 Mol des Diamins VI wurden zusammen mit 1,0 Mol des Dihalogenids V und 2,2 Mol Natriumcarbonat in 300 ml Butanol 6 Stunden auf Rückflußtemperatur erhitzt. Sodann wurde abgekühlt, filtriert und das Filtrat am Rotationsverdampfer eingeengt. Aus dem verbleibenden Rückstand wurde die Verbindung der Formel II durch fraktionierte Destillation gewonnen.
B) Ausgehend von einem Amin der Formel III und einer Verbindung der Formel IV.
   B1: 0,5 Mol einer Dichloralkanverbindung der Formel IV wurden zusammen mit 1 Mol eines Amins der Formel III und 1,1 Mol Natriumcarbonat in 200 ml Butanol 6 Stunden auf Rückflußtemperatur erhitzt. Nach dem Abkühlen des Reaktionsgemisches wurde von den ausgefallenen anorganischen Salzen abfiltriert und das Filtrat am Rotationsverdampfer eingeengt. Aus dem verbleibenden Rückstand wurde anschließend die Verbindung der Formel II durch fraktionierte Destillation isoliert.
   B2: Zu einer Lösung von 3,2 Mol eines Amins der Formel III in 300 ml Benzol wurden 0,8 Mol eines Dibromalkans der Formel IV so zugetropft, daß die Lösung am Sieden blieb. Nach beendeter Zugabe wurde noch eine Stunde unter Rückfluß erhitzt. Sodann wurde das Reaktionsgemisch abgekühlt, filtriert und das Filtrat eingeengt. Aus dem verbleibenden Rückstand wurde die Verbindung der Formel II durch fraktionierte Destillation gewonnen.
C) Ausgehend von einem Amin der Formel III und einem Diol der Formel IV.
   In einem Autoklaven wurden 1 Mol eines Amins der Formel III und 0,4 Mol eines Diols der Formel IV in 100 ml Dioxan mit 2 g Kupferchromit unter einem Wasserstoffdruck von 100 Atmosphären 40 Stunden lang auf 250 °C erhitzt. Anschließend wurde das Reaktionsgemisch filtriert und aus dem Filtrat wurde die Verbindung der Formel II durch fraktionierte Destillation gewonnen.

Nach den vorstehenden allgemeinen Arbeitsvorschriften wurden die folgenden in Tabelle 2 angegebenen Verbindungen der Formel II hergestellt.

**Tabelle 2**

| Ausgangsverbindungen der Formel II | | | |
|---|---|---|---|
| Nr. | Substanzname | hergestelt analog Vorschrift | Ausbeute |
| A 1 | Dimorpholinoethan | B I | 62 % |
| | | A | 22 % |
| A 2 | Dimorpholinopropan | A | 42 % |
| | | B I | 66 % |
| | | B II | 77 % |
| A 3 | Dimorpholinobutan | B I | 40 % |
| A 4 | Dimorpholinopentan | A | 13 % |
| | | B II | 46 % |
| | | C | 14 % |
| A 5 | Dimorpholinoheptan | B II | 23 % |
| A 6 | Dimorpholinooctan | B II | 18 % |
| A 7 | Dimorpholinohexan | B II | 72 % |
| A 8 | Dipiperidinoethan | B I | 35 % |
| | | B II | 30 % |
| A 9 | Dipiperidinopropan | B II | 89 % |
| A 10 | Dipiperidinobutan | B I | 18 % |
| | | B II | 20 % |
| A 11 | Dipiperidinopentan | C | 10 % |
| | | B II | 7 % |
| A 12 | Dipiperidinohexan | B II | 49 % |
| A 13 | Diperidinoheptan | B II | 26 % |
| A 14 | Dipiperidinooctan | B II | 17 % |

### Beispiel I:

Herstellung einer Perfluordecalin enthaltenden stabilisierten wäßrigen Emulsion.

2 g Perfluordecalin und 0,1 g Perfluorodimorpholinobutan wurden zu 0,3 g des Emulgators Pluronic^{R} F68 (=Ethylenoxid/Propylenoxid-Blockpolymeres, mittleres Molekulargewicht 8300, Hersteller Firma Wyandotte) gegeben und das Gemisch wurde mit Wasser auf ein Gesamtvolumen von 10 ml aufgefüllt. Es wurde 2 Minuten mit einem Ultraschallhomogenisator, (Modell Labsonic^{R} 2000 mit Standardsonde der Fa. Braun, Melsungen) bei Output 170 homogenisiert. Die entstandene Emulsion hatte eine mittlere Kolloidteilchengröße von ca. 100 - 150 nm, welche bei Lagerung bei +4 °C über mehrere Wochen kaum anstieg.

### Beispiel II:

Eine als Blutersatzmittel oder Perfusionsmedium geeignete Perfluorodecalin enthaltende wäßrige Emulsion.

| Zusammensetzung: | |
|---|---|
| Perfluorodecalin | 190 g/l |
| Perfluorodimorpholinobutan | 10 g/l |
| Emulgator (Pluronic^{R} F68) | 27 g/l |
| Glycerin | 8 g/l |
| Eiweißphospholipide | 4 g/l |
| Hydroxyäthylstärke (mittleres Molekulargewicht 200 000) | 30 g/l |
| Na⁺ | 128,0 mMol/l |
| K⁺ | 4,5 mMol/l |
| Ca⁺⁺ | 2,5 mMol/l |
| Mg⁺⁺ | 2,1 mMol/l |
| Cl⁻ | 116,0 mMol/l |
| HCO₃⁻ | 25 mMol/l |
| Glucose | 10,0 mMol/l |
| Steriles destilliertes Wasser | ad 1 l |

Die vorstehend angegebenen Bestandteile wurden in an sich bekannter Weise homogenisiert.

### Beispiel III:

Herstellung einer Perfluorocyclohexylmorpholin enthaltenden stabilisierten wäßrigen Emulsion.

200 g eines Perfluorocyclohexylmorpholin und Perfluoro-n-hexylmorpholin im Verhältnis 2 : 1 enthaltenden Gemisches (hergestellt analog Beispiel 1 durch elektrochemische Fluorierung von durch Umsetzung von Morpholin mit Cyclohexanon erhaltenem Morpholinocyclohexan-(1); Siedepunkt 147,5 - 148,5 °C) und 10 g Perfluorodimorpholinobutan wurden zu 35 g des Emulgators Eidotterlecithin gegeben und das Gemisch mit Wasser auf ein Gesamtvolumen von 1000 ml aufgefüllt.

Es wurde in einem Homogenisator der Fa. Gaulin, Typ Lab 60/60 - 10 TBS mit einem Druck von 700 bar in 5 Durchläufen homogenisiert. Die entstandene Emulsion hatte eine Kolloidteilchengröße von 100 - 150 nm, welche bei Lagerung bei +4 °C über mehrere Wochen kaum anstieg.

### Beispiel IV:

Stabilitätsvergleich einer mit Perfluorodimorpholinopropan stabilisierten wäßrigen Perfluorodecalin-Emulsion bei verschiedenen Lagertemperaturen.

Es wurde eine Emulsion aus 20 % (G/V) Perfluorodecalin, 1 % (G/V) Perfluorodimorpholinopropan und 4 % (G/V) Emulgator (Pluronic^{R} F 68) in Wasser hergestellt. Hierzu wurden die Bestandteile 2 Minuten lang mit einem Ultraschallhomogenisator (Modell Labsonic^{R} 2000, mit Standardsonde der Fa. Braun, Melsungen) bei Output 170 homogenisiert.

Anschließend wurde die mittlere Kolloidteilchengröße mit Hilfe eines Autosizers (Malvern Autosizer IIc mit Automeasure Softwareprogramm 4,1) bestimmt. Dann wurden ein Teil der Emulsion bei 6 °C und ein weiterer Teil bei 37 °C Lagertemperatur während 13 Tagen gelagert und die Teilchengrößenbestimmungen in beiden Teilen mehrmals während der Lagerdauer durchgeführt. Die Ergebnisse sind in der nachfolgenden Tabelle I wiedergegeben.

**Tabelle I**

| Lagerdauer T in Tagen | mittlere Kolloidteilchengröße D in nm bei Lagertemperatur | | Dt/Do 6 °C | Dt/Do 37 °C |
|---|---|---|---|---|
| | 6 °C | 37 °C | | |
| 0 | 194,3 | 194,3 | 1 | |
| 1 | 265,1 | 254,2 | 1,364 | 1,309 |
| 8 | 299,6 | 286,0 | 1,542 | 1,472 |
| 10 | 289,9 | 270,0 | 1,477 | 1,39 |
| 13 | 286,5 | 300,3 | 1,475 | 1,546 |

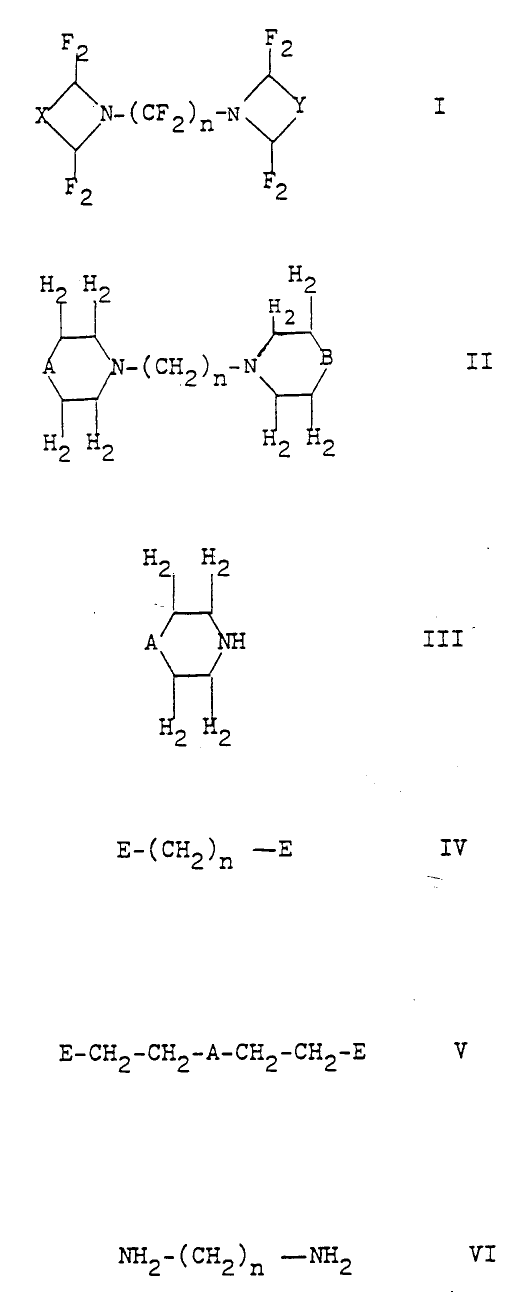

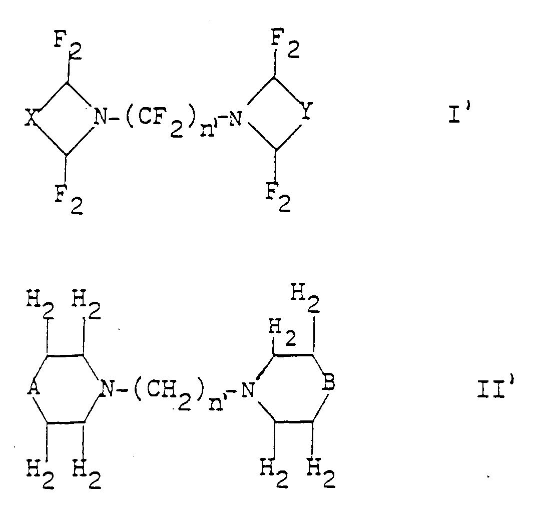

## Patentansprüche (Patentansprüche für folgende(n) Vertragsstaat(en): AT, BE, CH, DE, DK, FR, GB, IT, LI, NL, SE)

1. Verwendung von perfluorierten heterocyclischen Verbindungen der allgemeinen Formel I, 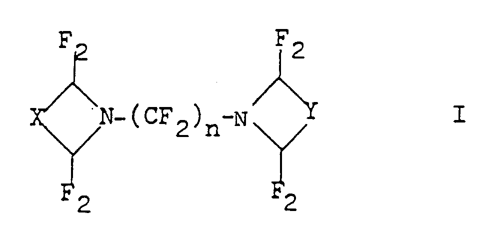 worin
n für 2 - 8 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemischen als emulsionsstabilisierende Zusätze in medizinisch verwendbaren wäßrigen Emulsionen von zur Verwendung in Blutersatzmitteln geeigneten gegebenenfalls Heteroatome enthaltenden Perfluorkohlenstoffen.

2. Zur Herstellung von medizinisch verwendbaren wäßrigen Emulsionen geeignetes Gemisch physiologisch verträglicher gegebenenfalls Heteroatome enthaltender Perfluorkohlenstoffe, dadurch gekennzeichnet, daß es zur Verwendung als Sauerstoffüberträger in Blutersatzmitteln geeignete gegebenenfalls Heteroatome enthaltende Perfluorkohlenstoffe und perfluorierte heterocyclische Verbindungen der allgemeinen Formel I, 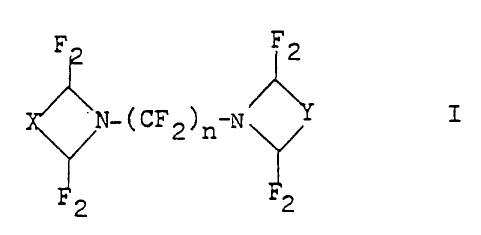 worin
n für 2 - 8 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemische enthält.

3. Gemische gemäß Anspruch 2, dadurch gekennzeichnet, daß sie zwischen 1 und 20, vorzugsweise 2,5 und 10 Gew.-%, an Verbindungen der Formel I enthalten.

4. Gemische gemäß Anspruch 2 und 3, dadurch gekennzeichnet, daß sie Verbindungen der Formel I enthalten, worin n für 3 - 6, vorzugsweise 3 oder 4, steht.

5. Gemische gemäß Anspruch 2, dadurch gekennzeichnet, daß die als Sauerstoffüberträger geeigneten gegebenenfalls Heteroatome enthaltenden Perfluorkohlenstoffe bei Körpertemperatur flüssige Substanzen mit Siedepunkten im Bereich von 130 - 200 °C sind.

6. Gemische gemäß Anspruch 2, dadurch gekennzeichnet, daß die als Sauerstoffüberträger geeigneten gegebenenfalls Heteroatome enthaltenden Perfluorkohlenstoffe Molekulargewichte im Bereich von 450 - 600 besitzen.

7. Gemische gemäß Anspruch 5 oder 6, dadurch gekennzeichnet, daß die als Sauerstoffüberträger geeigneten Substanzen Substanzen mit einer Verweilhalbwertzeit im Körper von einer Woche bis zu einem Monat sind.

8. Gemische gemäß Anspruch 2, dadurch gekennzeichnet, daß sie als Sauerstoffüberträger geeignete Substanz Perfluorodecalin enthalten.

9. Gemische gemäß Anspruch 2, dadurch gekennzeichnet, daß sie als Sauerstoffüberträger geeignete Substanzen Perfluoro-N-cyclohexylmorpholin oder ein Gemisch aus Perfluoro-N-cyclohexylmorpholin und Perfluoro-N-n-hexylmorpholin enthalten.

10. Medizinisch verwendbare wäßrige Emulsionen, dadurch gekennzeichnet, daß sie in einer physiologisch verträglichen wäßrigen Phase ein Gemisch von physiologisch verträglichen gegebenenfalls Heteroatome enthaltenden Perfluorkohlenstoffen, welches zur Verwendung als Sauerstoffüberträger in Blutersatzmitteln geeignete gegebenenfalls Heteroatome enthaltende Perfluorkohlenstoffe und perfluorierte heterocyclische Verbindungen der allgemeinen Formel I, 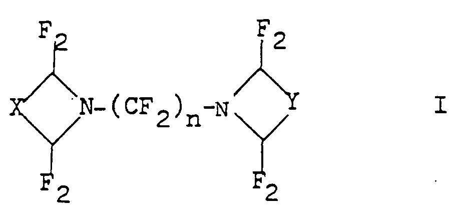 worin
n für 2 - 8 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemische enthält, emulgiert enthalten.

11. Medizinisch verwendbare Emulsionen gemäß Anspruch 10, dadurch gekennzeichnet, daß sie einen physiologisch verträglichen Emulgator enthalten,

12. Emulsionen gemäß Anspruch 10 und 11, dadurch gekennzeichnet, daß sie Gemische von physiologisch verträglichen gegebenenfalls Heteroatome enthaltenden Perfluorkohlenstoffen in Konzentrationen von 5 - 50, insbesondere 15 - 25, g pro 100 ml Emulsion enthalten.

13. Emulsionen gemäß Anspruch 10 bis 12, dadurch gekennzeichnet, daß das Gemisch an physiologisch verträglichen gegebenenfalls Heteroatome enthaltenden Perfluorokohlenstoffen von 1 bis 20, vorzugsweise 2,5 - 10 Gew.-%, bezogen auf das Perfluorkohlenstoffgemisch an Verbindungen der Formel I enthält,

14. Perfluorierte heterocyclische Verbindungen der allgemeinen Formel I', 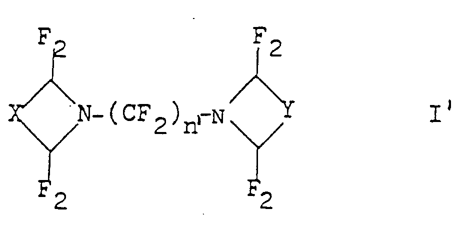 worin
n' für 4 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemische,

15. Verfahren zur Herstellung von perfluorierten heterocyclischen Verbindungen der allgemeinen Formel I' 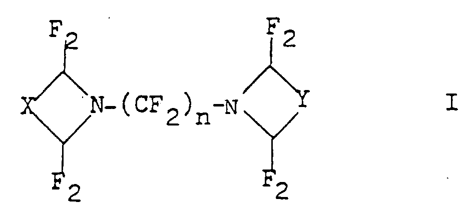 worin
n' für 4 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemischen,
dadurch gekennzeichnet, daß man
a) eine Lösung von Verbindungen der allgemeinen Formel II' 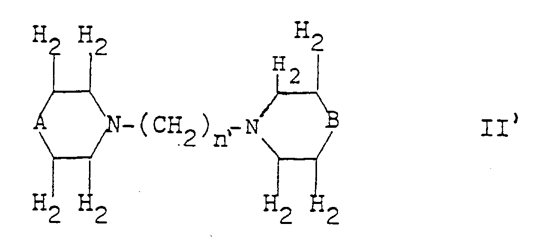 worin
n' obige Bedeutung besitzt,
A Sauerstoff oder eine -CH₂-Gruppe bedeutet, und
B Sauerstoff oder eine -CH₂-Gruppe bedeutet,
in flüssigem Fluorwasserstoff elektrolysiert und ein die perfluorierten Verbindungen der Formel I neben nur partiell fluorierten Nebenprodukten enthaltendes rohes Reaktionsprodukt abtrennt,
b) das rohe Reaktionsprodukt mit einer Alkali- oder Erdalkalimetallbase, insbesondere einem Alkali- oder Erdalkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur behandelt,
c) die Verbindungen der Formel I' oder deren Gemische aus einem in Verfahrensstufe b) erhaltenem Reaktionsgemisch isoliert, und
d) gewünschtenfalls erhaltene Isomerengemische aus Verbindungen der allgemeinen Formel I', worin X und/oder Y eine -CF₂-CF₂-CF₂-Gruppe bedeuten und n' obige Bedeutung besitzt, und dazu isomeren Verbindungen der Formel I', worin X und/oder Y eine -CF(CF₃)-CF₂-Gruppe bedeuten, in die einzelnen Isomeren auftrennt.

## Patentansprüche (Patentansprüche für folgende(n) Vertragsstaat(en): ES)

1. Verfahren zur Stabilisierung von medizinisch verwendbaren wäßrigen Emulsionen von zur Verwendung in Blutersatzmitteln geeigneten gegebenenfalls Heteroatome enthaltenden Perfluorkohlenstoffen, dadurch gekennzeichnet, daß man perfluorierte heterocyclische Verbindungen der allgemeinen Formel I, 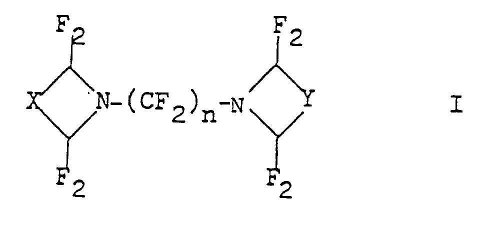 worin
n für 2-8 steht,
X eine -CF₂-0-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemische als emulsionsstabilisierende Zusätze beifügt.

2. Verfahren zur Herstellung eines zur Herstellung von medizinisch verwendbaren wäßrigen Emulsionen geeigneten Gemisches physiologisch verträglicher gegebenenfalls Heteroatome enthaltender Perfluorkohlenstoffe, dadurch gekennzeichnet, daß man zur Verwendung als Sauerstoffüberträger in Blutersatzmitteln geeignete gegebenenfalls Heteroatome enthaltende Perfluorkohlenstoffe mit perfluorierten heterocyclischen Verbindungen der allgemeinen Formel I 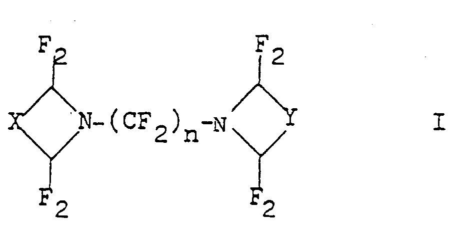 worin
n für 2-8 steht,
X eine -CF₂-O-CF₂-Gruppe, eine CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemischen mischt.

3. Verfahren gemäß Anspruch 2, dadurch gekennzeichnet, daß Gemische, welche zwischen 1 und 20, vorzugsweise 2,5 und 10 Gew.-%, an Verbindungen der Formel I enthalten, hergestellt werden.

4. Verfahren gemäß Anspruch 2 und 3, dadurch gekennzeichnet, daß Gemische, welche Verbindungen der Formel I enthalten, worin n für 3-6, vorzugsweise 3 oder 4, steht, hergestellt werden.

5. Verfahren gemäß Anspruch 2, dadurch gekennzeichnet, daß die als Sauerstoffüberträger geeigneten gegebenenfalls Heteroatome enthaltenden Perfluorkohlenstoffe bei Körpertemperatur flüssige Substanzen mit Siedepunkten im Bereich von 130-200 °C sind.

6. Verfahren gemäß Anspruch 2, dadurch gekennzeichnet, daß die als Sauerstoffüberträger geeigneten gegebenenfalls Heteroatome enthaltenden Perfluorkohlenstoffe Molekulargewichte im Bereich von 450-600 besitzen.

7. Verfahren gemäß Anspruch 5 oder 6, dadurch gekennzeichnet, daß die als Sauerstoffüberträger geeigneten Substanzen Substanzen mit einer Verweilhalbwertzeit im Körper von einer Woche bis zu einem Monat sind.

8. Verfahren gemäß Anspruch 2, dadurch gekennzeichnet, daß Gemische, welche als Sauerstoffüberträger geeignete Substanz Perfluorodecalin enthalten, hergestellt werden.

9. Verfahren gemäß Anspruch 2, dadurch gekennzeichnet, daß Gemische, welche als Sauerstoffüberträger geeignete Substanzen Perfluoro-N-cyclohexylmorpholin oder ein Gemisch aus Perfluoro-N-cyclohexylmorpholin und Perfluoro-N-n-hexylmorpholin enthalten, hergestellt werden.

10. Verfahren zur Herstellung von medizinisch verwendbaren wäßrigen Emulsionen, dadurch gekennzeichnet, daß eine physiologisch verträgliche wäßrige Phase mit einem Gemisch von physiologisch verträglichen gegebenenfalls Heteroatome enthaltenden Perfluorkohlenstoffen, welches zur Verwendung als Sauerstoffüberträger in Blutersatzmitteln geeignete gegebenenfalls Heteroatome enthaltende Perfluorkohlenstoffe und perfluorierte heterocyclische Verbindungen der allgemeinen Formel I, 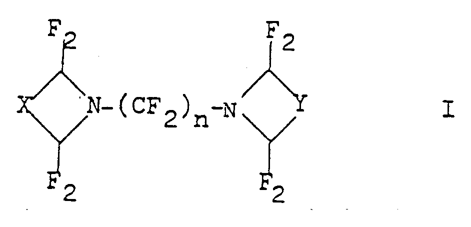 worin
n für 2-8 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemische enthält, emulgiert wird.

11. Verfahren gemäß Anspruch 10, dadurch gekennzeichnet, daß medizinisch verwendbare Emulsionen hergestellt werden, welche einen physiologisch verträglichen Emulgator enthalten.

12. Verfahren gemäß Anspruch 10 und 11, dadurch gekennzeichnet, daß Emulsionen, welche Gemische von physiologisch verträglichen gegebenenfalls Heteroatome enthaltenden Perfluorkohlenstoffen in Konzentrationen von 5-50, insbesondere 15-25 g pro 100 ml Emulsion enthalten, hergestellt werden.

13. Verfahren gemäß Anspruch 10 bis 12, dadurch gekennzeichnet, daß das Gemisch an physiologisch verträglichen gegebenenfalls Heteroatome enthaltenden Perfluorkohlenstoffen von 1 bis 20, vorzugsweise 2,5 bis 10 Gew.-%, bezogen auf das Perfluorkohlenstoffgemisch an Verbindungen der Formel I enthält.

14. Verfahren zur Herstellung von perfluorierten heterocyclischen Verbindungen der allgemeinen Formel I' 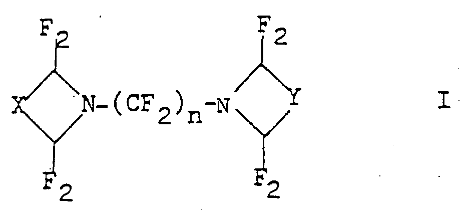 worin
n' für 4 steht,
X eine -CF₂-O-CF₂-Gruppe, eine -CF₂-CF₂-CF₂-Gruppe oder eine -CF(CF₃)-CF₂-Gruppe darstellt, und
Y eine -CF₂-O-CF₂-Gruppe, eine -CF(CF₃)-CF₂-Gruppe oder eine -CF₂-CF₂-CF₂-Gruppe darstellt,
oder deren Gemischen, dadurch gekennzeichnet, daß man
a) eine Lösung von Verbindungen der allgemeinen Formel II' 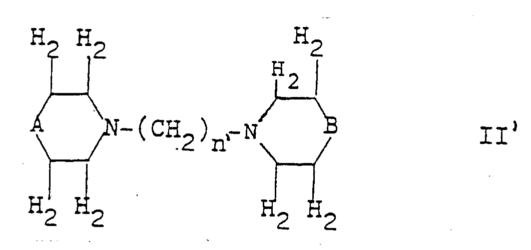 worin
n' obige Bedeutung besitzt,
A Sauerstoff oder eine -CH₂-Gruppe bedeutet, und
B Sauerstoff oder eine -CH₂-Gruppe bedeutet,
in flüssigem Fluorwasserstoff elektrolysiert und ein die perfluorierten Verbindungen der Formel I neben nur partiell fluorierten Nebenprodukten enthaltendes rohes Reaktionsprodukt abtrennt,
b) das rohe Reaktionsprodukt mit einer Alkali- oder Erdalkalimetallbase, insbesondere einem Alkali- oder Erdalkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur behandelt,
c) die Verbindungen der Formel I' oder deren Gemische aus einem in Verfahrensstufe b) erhaltenem Reaktionsgemisch isoliert, und
d) gewünschtenfalls erhaltene Isomerengemische aus Verbindungen der allgemeinen Formel I', worin X und/oder Y eine -CF₂-CF₂-CF₂-Gruppe bedeuten und n' obige Bedeutung besitzt, und dazu iomeren Verbindungen der Formel I', worin X und/oder Y eine -CF(CF₃)-CF₂-Gruppe bedeuten, in die einzelnen Isomeren auftrennt.

## Claims (Claims for the following Contracting State(s): AT, BE, CH, DE, DK, FR, GB, IT, LI, NL, SE)

1. The use of perfluorinated heterocyclic compounds of the general formula I, 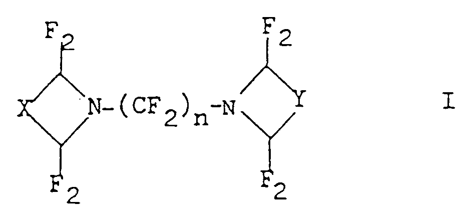 wherein
n stands for 2 - 8,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof as emulsion-stabilising additives in medically usable aqueous emulsions of perfluorocarbons, optionally containing hetero atoms, suitable for use in blood substitutes.

2. A mixture of physiologically acceptable perfluorocarbons, optionally containing hetero atoms, suitable for preparing medically usable aqueous emulsions, characterised in that it contains perfluorocarbons, optionally containing hetero atoms, suitable for use as oxygen carriers in blood substitutes, and perfluorinated heterocyclic compounds of the general formula I, 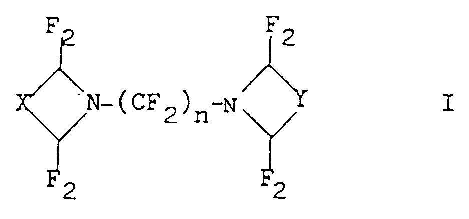 wherein
n stands for 2 - 8,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof.

3. Mixtures according to Claim 2, characterised in that they contain between 1 and 20, preferably 2.5 and 10, % by weight of compounds of formula I.

4. Mixtures according to Claims 2 and 3, characterised in that they contain compounds of formula I in which n stands for 3 - 6, preferably 3 or 4.

5. Mixtures according to Claim 2, characterised in that the perfluorocarbons, optionally containing hetero atoms, which are suitable as oxygen carriers are liquid substances at body temperature with boiling points in the range of 130 - 200°C.

6. Mixtures according to Claim 2, characterised in that the perfluorocarbons, optionally containing hetero atoms, which are suitable as oxygen carriers have molecular weights in the range of 450 - 600.

7. Mixtures according to Claim 5 or 6, characterised in that the substances which are suitable as oxygen carriers are substances having a half-life in the body of one week to one month.

8. Mixtures according to Claim 2, characterised in that they contain perfluorodecalin as the substance suitable as an oxygen carrier.

9. Mixtures according to Claim 2, characterised in that they contain perfluoro-N-cyclohexylmorpholine or a mixture of perfluoro-N-cyclohexylmorpholine and perfluoro-N-n-hexylmorpholine as the substances suitable as oxygen carriers.

10. Medically usable aqueous emulsions, characterised in that they contain in a physiologically acceptable aqueous phase a mixture of emulsified physiologically acceptable perfluorocarbons, optionally containing hetero atoms, which contains perfluorocarbons, optionally containing hetero atoms, suitable for use as oxygen carriers in blood substitutes, and perfluorinated heterocyclic compounds of the general formula I, 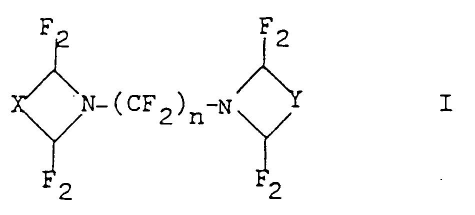 wherein
n stands for 2 - 8,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof.

11. Medically usable emulsions according to Claim 10, characterised in that they contain a physiologically acceptable emulsifier.

12. Emulsions according to Claims 10 and 11, characterised in that they contain mixtures of physiologically acceptable perfluorocarbons, optionally containing hetero atoms, in concentrations of 5 - 50, especially 15 - 25, g per 100 ml of emulsion.

13. Emulsions according to Claims 10 to 12, characterised in that the mixture of physiologically acceptable perfluorocarbons, optionally containing hetero atoms, contains from 1 to 20, preferably 2.5 - 10, % by weight, based on the perfluorocarbon mixture, of compounds of formula I.

14. Perfluorinated heterocyclic compounds of the general formula I', 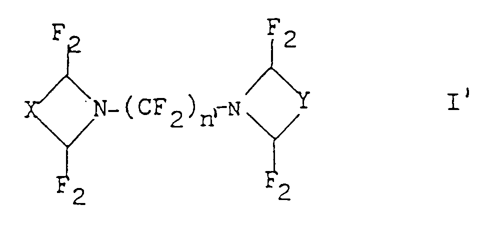 wherein
n' stands for 4,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof.

15. A method for the preparation of perfluorinated heterocyclic compounds of the general formula I', 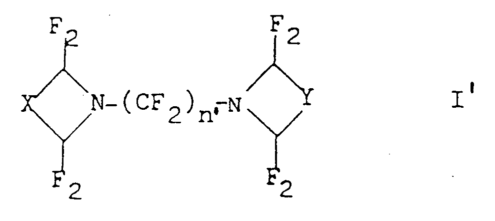 wherein
n' stands for 4,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof,
characterised in that
a) a solution of compounds of the general formula II', 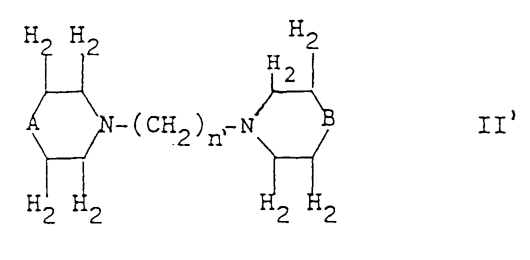 wherein
n' has the above meaning,
A represents oxygen or a -CH₂- group, and
B represents oxygen or a -CH₂- group,
is electrolysed in liquid hydrogen fluoride and a crude reaction product containing the perfluorinated compounds of formula I in addition to only partially fluorinated byproducts is separated off,
b) the crude reaction product is treated with an alkali metal or alkaline earth metal base, especially an alkali metal or alkaline earth metal hydroxide, in the presence of water and optionally a lower aliphatic primary or secondary amine, at an elevated temperature sufficient for the decomposition of only partially fluorinated byproducts,
c) the compounds of formula I' or their mixtures are isolated from a reaction mixture obtained in process step b), and
d) if desired, the resulting isomer mixtures of compounds of the general formula I', wherein X and/or Y represent a -CF₂-CF₂-CF₂- group and n' has the above meaning, and compounds of formula I' isomeric thereto, wherein X and/or Y represent a -CF(CF₃)-CF₂- group, are separated into the individual isomers.

## Claims (Claims for the following Contracting State(s): ES)

1. A method for stabilising medically usable aqueous emulsions of perfluorocarbons, optionally containing hetero atoms, suitable for use as oxygen carriers in blood substitutes, characterised in that perfluorinated heterocyclic compounds of the general formula I, 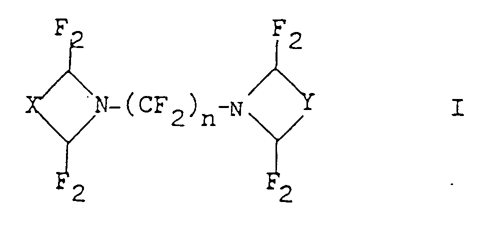 wherein
n stands for 2 - 8,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof are added as emulsion-stabilising additives.

2. A method for preparing a mixture of physiologically acceptable perfluorocarbons, optionally containing hetero atoms, suitable for preparing medically usable aqueous emulsions, characterised in that perfluorocarbons, optionally containing hetero atoms, suitable for use as oxygen carriers in blood substitutes, are mixed with perfluorinated heterocyclic compounds of the general formula I, 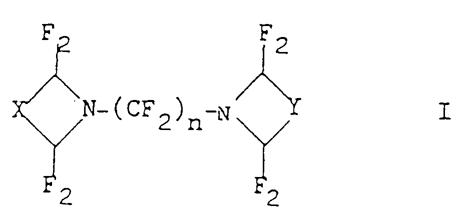 wherein
n stands for 2 - 8,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof.

3. A method according to Claim 2, characterised in that mixtures which contain between 1 and 20, preferably 2.5 and 10, % by weight of compounds of formula I are produced.

4. A method according to Claims 2 and 3, characterised in that mixtures which contain compounds of formula I in which n stands for 3 - 6, preferably 3 or 4 are produced.

5. A method according to Claim 2, characterised in that the perfluorocarbons, optionally containing hetero atoms, which are suitable as oxygen carriers are liquid substances at body temperature with boiling points in the range of 130 - 200°C.

6. A method according to Claim 2, characterised in that the perfluorocarbons, optionally containing hetero atoms, which are suitable as oxygen carriers have molecular weights in the range of 450 - 600.

7. A method according to Claim 5 or 6, characterised in that the substances which are suitable as oxygen carriers are substances having a half-life in the body of one week to one month.

8. A method according to Claim 2, characterised in that mixtures which contain perfluorodecalin as the substance suitable as an oxygen carrier are produced.

9. A method according to Claim 2, characterised in that mixtures which contain perfluoro-N-cyclohexylmorpholine or a mixture of perfluoro-N-cyclohexylmorpholine and perfluoro-N-n-hexylmorpholine as the substances suitable as oxygen carriers are produced.

10. A method for the preparation of medically usable aqueous emulsions, characterised in that a physiologically acceptable aqueous phase is emulsified with a mixture of physiologically acceptable perfluorocarbons, optionally containing hetero atoms, which contains perfluorocarbons, optionally containing hetero atoms, suitable for use as oxygen carriers in blood substitutes, and perfluorinated heterocyclic compounds of the general formula I, 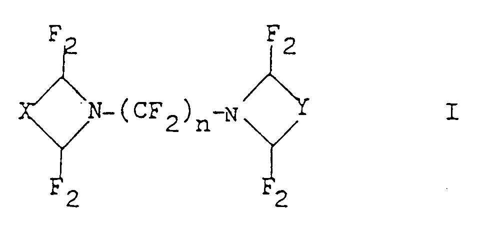 wherein
n stands for 2 - 8,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof.

11. A method according to Claim 10, characterised in that medically usable emulsions are produced which contain a physiologically acceptable emulsifier.

12. A method according to Claims 10 and 11, characterised in that emulsions are produced which contain mixtures of physiologically acceptable perfluorocarbons, optionally containing hetero atoms, in concentrations of 5 - 50, especially 15 - 25, g per 100 ml of emulsion.

13. A method according to Claims 10 to 12, characterised in that the mixture of physiologically acceptable perfluorocarbons, optionally containing hetero atoms, contains from 1 to 20, preferably 2.5 - 10, % by weight, based on the perfluorocarbon mixture, of compounds of formula I.

14. A method for the preparation of perfluorinated heterocyclic compounds of the general formula I', 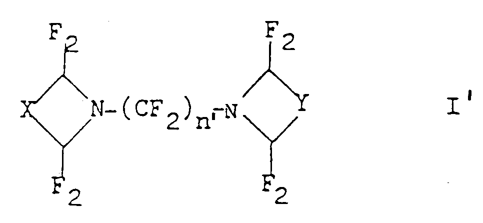 wherein
n' stands for 4,
X is a -CF₂-O-CF₂ group, a -CF₂-CF₂-CF₂ group or a -CF(CF₃)-CF₂ group, and
Y is a -CF₂-O-CF₂ group, a -CF(CF₃)-CF₂ group or a -CF₂-CF₂-CF₂ group,
or mixtures thereof, characterised in that
a) a solution of compounds of the general formula II', 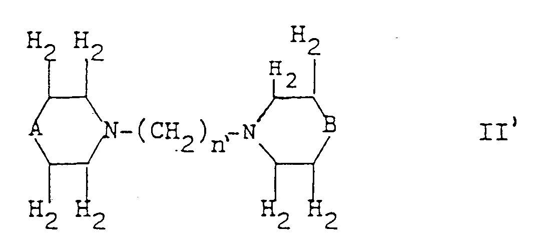 wherein
n' has the above meaning,
A represents oxygen or a -CH₂- group, and
B represents oxygen or a -CH₂- group,
is electrolysed in liquid hydrogen fluoride and a crude reaction product containing the perfluorinated compounds of formula I in addition to only partially fluorinated byproducts is separated off,
b) the crude reaction product is treated with an alkali metal or alkaline earth metal base, especially an alkali metal or alkaline earth metal hydroxide, in the presence of water and optionally a lower aliphatic primary or secondary amine, at an elevated temperature sufficient for the decomposition of only partially fluorinated byproducts,
c) the compounds of formula I' or their mixtures are isolated from a reaction mixture obtained in process step b), and
d) if desired, the resulting isomer mixtures of compounds of the general formula I', wherein X and/or Y represent a -CF₂-CF₂-CF₂- group and n' has the above meaning, and compounds of formula I' isomeric thereto, wherein X and/or Y represent a -CF(CF₃)-CF₂- group, are separated into the individual isomers.

## Revendications (Revendications pour l'(les) Etat(s) contractant(s) suivant(s): AT, BE, CH, DE, DK, FR, GB, IT, LI, NL, SE)

1. Utilisation de composés hétérocycliques perfluorés de formule générale I 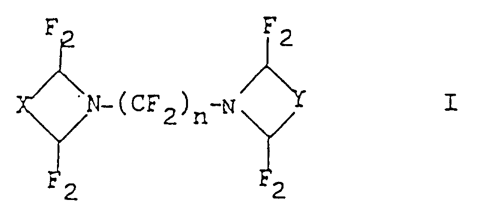 dans laquelle
n représente 2 à 8,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou de mélanges de ceux-ci, en tant qu'additifs stabilisateurs d'émulsion dans des émulsions aqueuses, utilisables en médecine, de perfluorocarbones contenant éventuellement des hétéroatomes, appropriés à l'utilisation dans des succédanés de sang.

2. Mélange de perfluorocarbones physiologiquement acceptables, contenant éventuellement des hétéroatomes, approprié à la préparation d'émulsions aqueuses utilisables en médecine, caractérisé en ce qu'il contient des perfluorocarbones, contenant éventuellement des hétéroatomes, appropriés à l'utilisation comme agents de transfert d'oxygène dans des succédanés de sang, et des composés hétérocycliques perfluorés de formule générale I 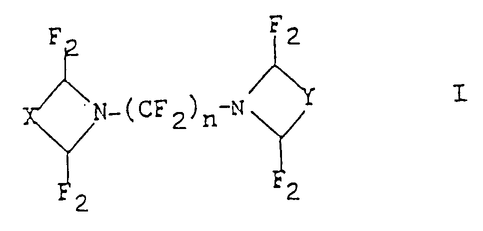 dans laquelle
n représente 2-8,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci.

3. Mélanges selon la revendication 2, caractérisés en ce qu'ils contiennent entre 1 et 20, de préférence entre 2,5 et 10 % en poids de composés de formule I.

4. Mélanges selon les revendications 2 et 3, caractérisés en ce qu'ils contiennent des composés de formule I dans lesquels n va de 3 à 6, de préférence est 3 ou 4.

5. Mélanges selon la revendication 2, caractérisés en ce que les perfluorocarbones contenant éventuellement des hétéroatomes, qui sont appropriés comme agents de transfert d'oxygène, sont des substances liquides à la température du corps, à points d'ébullition dans la plage de 130 à 200°C.

6. Mélanges selon la revendication 2, caractérisés en ce que les perfluorocarbones contenant éventuellement des hétéroatomes, qui sont appropriés en tant qu'agents de transfert d'oxygène, ont des masses moléculaires dans la plage allant de 450 à 600.

7. Mélanges selon la revendication 5 ou 6, caractérisés en ce que les substances appropriées en tant qu'agents de transfert d'oxygène sont des substances ayant une période de séjour dans l'organisme allant d'une semaine à un mois.

8. Mélanges selon la revendication 2, caractérisés en ce que, comme substance appropriée en tant qu'agent de transfert d'oxygène, ils contiennent de la Perfluorodécaline.

9. Mélanges selon la revendication 2, caractérisés en ce que, comme substances appropriées en tant qu'agent de transfert d'oxygène, ils contiennent de la perfluoro-N-cyclohexylmorpholine ou un mélange de perfluoro-N-cyclohexylmorpholine et de perfluoro-N-n-hexylmorpholine.

10. Emulsions aqueuses utilisables en médecine, caractérisées en ce qu'elles contiennent, émulsionné dans une phase aqueuse physiologiquement acceptable, un mélange de perfluorocarbones physiologiquement acceptables, contenant éventuellement des hétéroatomes, qui contient des perfluorocarbones, contenant éventuellement des hétéroatomes, appropriés à l'utilisation en tant qu'agents de transfert d'oxygène dans des succédanés de sang, et des composés hétérocycliques perfluorés de formule générale I 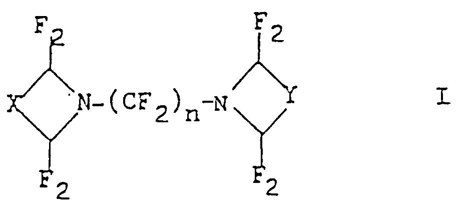 dans laquelle
n représente 2-8,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci.

11. Emulsions utilisables en médecine selon la revendication 10, caractérisées en ce qu'elles contiennent un émulsifiant physiologiquement acceptable.

12. Emulsions selon les revendications 10 et 11, caractérisées en ce qu'elles contiennent des mélanges de perfluorocarbones physiologiquement acceptables, contenant éventuellement des hétéroatomes, à des concentrations de 5 à 50, en particulier de 15 à 25 g pour 100 ml d'émulsion.

13. Emulsions selon les revendications 10 à 12, caractérisées en ce que le mélange de perfluorocarbones physiologiquement acceptables, contenant éventuellement des hétéroatomes, contient de 1 à 20, de préférence de 2,5 à 10 % en poids de composés de formule I, par rapport au mélange de perfluorocarbones.

14. Composés hétérocycliques perfluorés de formule générale I', 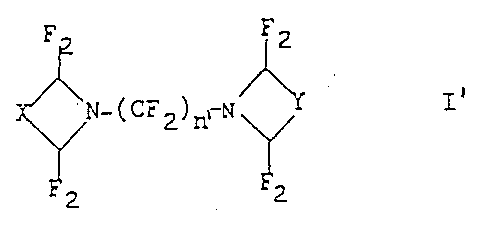 dans laquelle
n' représente 4,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci.

15. Procédé de préparation de composés hétérocycliques perfluorés de formule générale I' 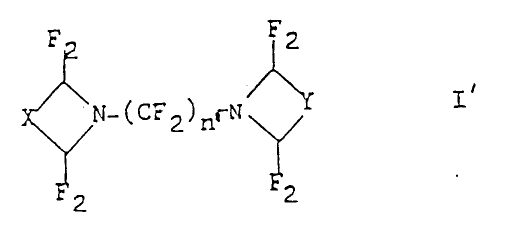 dans laquelle
n' représente 4,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci,
caractérisé en ce que
a) on électrolyse dans de l'acide fluorhydrique liquide une solution de composés de formule générale II' 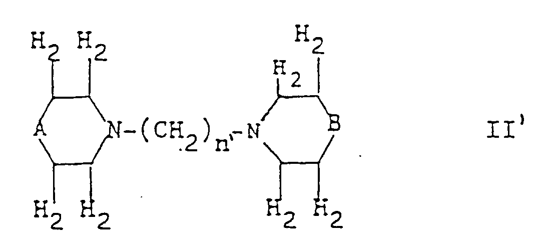 dans laquelle
n' a la signification donnée plus haut,
A représente un atome d'oxygène ou un groupe -CH₂-, et
B représente un atome d'oxygène ou un groupe -CH₂-, et on sépare un produit de réaction brut contenant les composés perfluorés de formule I en plus de produits secondaires seulement partiellement fluorés,
b) on traite le produit de réaction brut par une base dérivée d'un métal alcalin ou alcalino-terreux, en particulier un hydroxyde de métal alcalin ou alcalino-terreux, en présence d'eau et éventuellement d'une amine aliphatique primaire ou secondaire, à une température suffisamment élevée pour la décomposition des produits secondaires seulement partiellement fluorés,
c) on isole les composés de formule I' ou des mélanges de ceux-ci à partir d'un mélange réactionnel obtenu dans l'étape b) du procédé, et
d) si on le désire, on sépare en isomères individuels des mélanges d'isomères des composés de formule générale I' dans lesquels X et/ou Y représente(nt) un groupe -CF₂-CF₂-CF₂ et n' a la signification donnée ci-dessus, ainsi que les composés isomères de formule I' dans lesquels X et/ou Y représente(nt) un groupe -CF(CF₃)-CF₂-.

## Revendications (Revendications pour l'(les) Etat(s) contractant(s) suivant(s): ES)

1. Procédé de stabilisation d'émulsions aqueuses, utilisables en médecine, de perfluorocarbones contenant éventuellement des hétéroatomes, appropriés à l'utilisation dans des succédanés de sang, caractérisé en ce que l'on ajoute, en tant qu'additifs stabilisateurs d'émulsion, des composés hétérocycliques perfluorés de formule générale I 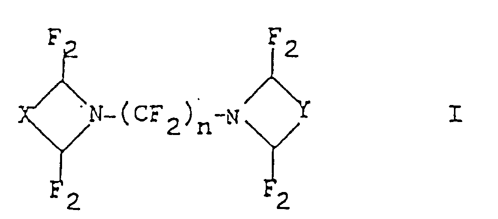 dans laquelle
n représente 2 à 8,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci.

2. Procédé de préparation d'un mélange de perfluorocarbones physiologiquement acceptables, contenant éventuellement des hétéroatomes, approprié à la préparation d'émulsions aqueuses utilisables en médecine, caractérisé en ce que l'on mélange des perfluorocarbones, contenant éventuellement des hétéroatomes, appropriés à l'utilisation comme agents de transfert d'oxygène dans des succédanés de sang, avec des composés hétérocycliques perfluorés de formule générale I 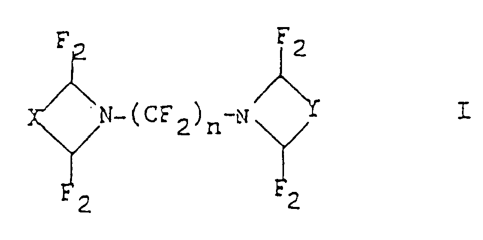 dans laquelle
n représente 2 à 8,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci.

3. Procédé selon la revendication 2, caractérisé en ce que l'on prépare des mélanges qui contiennent entre 1 et 20, de préférence entre 2,5 et 10 % en poids de composés de formule I.

4. Procédé selon la revendication 2, caractérisé en ce que l'on prépare des mélanges qui contiennent des composés de formule I dans lesquels n représente de 3 à 6, de préférence 3 ou 4.

5. Procédé selon la revendication 2, caractérisé en ce que les perfluorocarbones contenant éventuellement des hétéroatomes, qui sont appropriés comme agents de transfert d'oxygène, sont des substances liquides à la température du corps, à points d'ébullition dans la plage de 130 à 200°C.

6. Procédé selon la revendication 2, caractérisé en ce que les perfluorocarbones contenant éventuellement des hétéroatomes, qui sont appropriés en tant qu'agents de transfert d'oxygène, ont des masses moléculaires dans la plage allant de 450 à 600.

7. Procédé selon la revendication 5 ou 6, caractérisé en ce que les substances appropriées en tant qu'agents de transfert d'oxygène sont des substances ayant une période de séjour dans l'organisme allant d'une semaine à un mois.

8. Procédé selon la revendication 2, caractérisé en ce que l'on prépare des mélanges qui contiennent, comme substance appropriée en tant qu'agent de transfert d'oxygène, de la Perfluorodécaline.

9. Procédé selon la revendication 2, caractérisé en ce que l'on prépare des mélanges qui contiennent, comme substances appropriées en tant qu'agent de transfert d'oxygène, de la perfluoro-N-cyclohexylmorpholine ou un mélange de perfluoro-N-cyclohexylmorpholine et de perfluoro-N-n-hexylmorpholine.

10. Procédé de préparation d'émulsions aqueuses utilisables en médecine, caractérisé en ce qu'on émulsionne une phase aqueuse physiologiquement acceptable avec un mélange de perfluorocarbones physiologiquement acceptables, contenant éventuellement des hétéroatomes, qui contient des perfluorocarbones, contenant éventuellement des hétéroatomes, appropriés à l'utilisation en tant qu'agents de transfert d'oxygène dans des succédanés de sang, et des composés hétérocycliques perfluorés de formule générale I 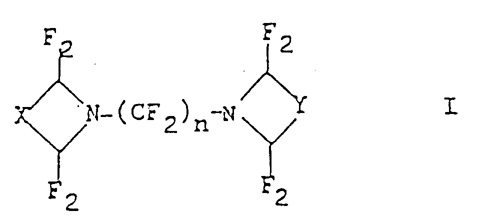 dans laquelle
n représente 2-8,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou des mélanges de ceux-ci.

11. Procédé selon la revendication 10, caractérisé en ce que l'on prépare des émulsions, utilisables en médecine, qui contiennent un émulsifiant physiologiquement acceptable.

12. Procédé selon les revendications 10 et 11, caractérisé en ce que l'on prépare des émulsions qui contiennent des mélanges de perfluorocarbones physiologiquement acceptables, contenant éventuellement des hétéroatomes, à des concentrations de 5 à 50, en particulier de 15 à 25 g pour 100 ml d'émulsion.

13. Procédé selon les revendications 10 à 12, caractérisé en ce que le mélange de perfluorocarbones physiologiquement acceptables, contenant éventuellement des hétéroatomes, contient de 1 à 20, de préférence de 2,5 à 10 % en poids de composés de formule I, par rapport au mélange de perfluorocarbones.

14. Procédé de préparation de composés hétérocycliques perfluorés de formule générale I', 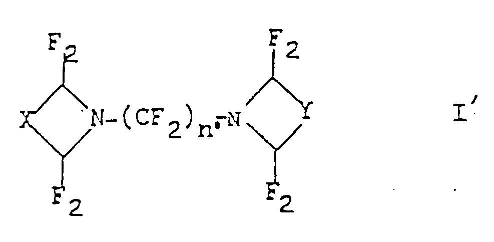 dans laquelle
n' représente 4,
X représente un groupe -CF₂-O-CF₂-, un groupe -CF₂-CF₂-CF₂- ou un groupe -CF(CF₃)-CF₂-, et
Y représente un groupe -CF₂-O-CF₂-, un groupe -CF(CF₃)-CF₂- ou un groupe -CF₂-CF₂-CF₂-,
ou de mélanges de ceux-ci, caractérisé en ce que
a) on électrolyse dans de l'acide fluorhydrique liquide une solution de composés de formule générale II' 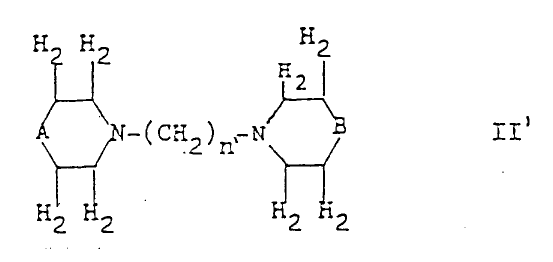 dans laquelle
n' a la signification donnée plus haut,
A représente un atome d'oxygène ou un groupe -CH₂-, et
B représente un atome d'oxygène ou un groupe -CH₂-, et on sépare un produit de réaction brut contenant les composés perfluorés de formule I en plus de produits secondaires seulement partiellement fluorés,
b) on traite le produit de réaction brut par une base dérivée d'un métal alcalin ou alcalino-terreux, en particulier un hydroxyde de métal alcalin ou alcalino-terreux, en présence d'eau et éventuellement d'une amine aliphatique primaire ou secondaire, à une température suffisamment élevée pour la décomposition des produits secondaires seulement partiellement fluorés,
c) on isole les composés de formule I' ou des mélange de ceux-ci à partir d'un mélange réactionnel obtenu dans l'étape b) du procédé, et
d) si on le désire on sépare en isomères individuels des mélanges d'isomères des composés de formule générale I' dans lesquels X et/ou Y représente(nt) un groupe -CF₂-CF₂-CF₂ et n' a la signification donnée ci-dessus, ainsi que les composés isomères de formule I' dans lesquels X et/ou Y représente(nt) un groupe -CF(CF₃)-CF₂-.
